(19) Europäisches Patentamt
European Patent Office
Office européen des brevets

(11) **EP 0 819 667 A2**

## (12) EUROPEAN PATENT APPLICATION

(43) Date of publication:
21.01.1998 Bulletin 1998/04

(51) Int Cl.$^6$: **C07C 15/14**, B41M 5/165

(21) Application number: **97305380.4**

(22) Date of filing: **18.07.1997**

(84) Designated Contracting States:
**AT BE CH DE DK ES FI FR GB GR IE IT LI LU MC NL PT SE**
Designated Extension States:
**AL LT LV RO SI**

(30) Priority: **19.07.1996 US 684500**

(71) Applicant: **KOCH INDUSTRIES, INC.**
**Wichita Kansas 67220 (US)**

(72) Inventors:
• **James, Dustin K.**
**Wichita, Kansas 67206 (US)**

• **Harding, Mary M.**
**Wichita, Kansas 67210 (US)**
• **Komin, Andrew P.**
**Wichita, Kansas 67206 (US)**

(74) Representative: **Perry, Robert Edward**
**GILL JENNINGS & EVERY**
**Broadgate House**
**7 Eldon Street**
**London EC2M 7LH (GB)**

(54) **Processes for production of mono- and/or di- sec. butylbiphenyl and products obtainable therefrom**

(57) Processes are provided for the production of sec-butylbiphenyl and di-sec-butylbiphenyl products which are useful as low odor dye solvents in carbonless paper systems. The products are formed by alkylation of biphenyl and butene in the presence of a Lewis acid catalyst complex or a zeolite catalyst such as Y, USY mordenite, and ZSM-12 zeolites. The Lewis acid catalyst complex uses a Lewis acid, an aromatic hydrocarbon and a hydrogen halide or alkyl halide, with more than about 50 mole % of the alkyl group substituents on the aromatic hydrocarbon being methyl groups. Feedstocks containing biphenyl, sec-butylbiphenyl and di-sec-butylbiphenyl may also be transalkylated using the Lewis acid catalyst complex or zeolite catalyst to obtain the desired low odor sec-butylbiphenyl and di-sec-butylbiphenyl product.

## Description

## Background of the Invention

This invention relates in general to processes for producing solvents such as for use in solubilizing dyes used in the production of carbonless paper. The invention also relates to the solvents produced by such processes, microcapsules containing a dye solubilized by such solvents, and pressure sensitive marking and recording material coated with such microcapsules.

Butenylated biphenyl products comprising primarily sec-butylbiphenyl and di-sec-butybiphenyl and produced by alkylation of biphenyl with butene are used as dye solvents in carbonless paper systems and in other functional fluid areas. One physical property of butenylated biphenyl which has sometimes limited its use is its odor. Although butenylated biphenyl has a low odor, somewhat lower than existing dye solvents for carbonless paper systems at the time of its development, it nonetheless has an odor. Because of the subsequent development of lower odor dye solvents, butenylated biphenyl products are now less favorable choices as dye solvents despite their other beneficial physical properties such as dye solvency and viscosity.

One process for the production of butenylated biphenyl products is disclosed in U.S. Patent No. 4,287,074 to Earhart et al. In that patent, the alkylation takes place in the presence of an aluminum chloride catalyst with a preferred operating temperature between 121°C to 246°C. It is stated in that patent that temperatures below 70°C would not be practical or commercially efficient. Operation at the preferred conditions is said to avoid the production of unwanted t-butylbiphenyl isomers. The patent does not disclose the odor of the product produced at a temperature lower than the preferred range, nor does it disclose the presence of isobutylbiphenyl isomers at the higher temperature range. However, the analytic techniques in use at the time were not generally sufficient to separate the isobutylbiphenyl isomers from the sec-butylbiphenyl isomers. Improvements in analytical techniques have led to the detection and measurement of components which were not separated or detected in the past. Products which were once thought to have been pure have been found to be less pure due to advances in gas chromatography, liquid chromatography and the like.

A process for the production of sec-butylbenzene was disclosed in U.S. Patent No. 5,191,136 to Takahashi et al. In that patent, a temperature range of 80°C to 150°C, preferably 90°C to 120°C, is said to be necessary to produce sec-butylbenzene which is free of isobutylbenzene. A specific aluminum chloride catalyst complex concentration and a specific reaction time are also claimed to be necessary for the process to be successful.

There are also numerous disclosures concerning the use of zeolites in the alkylation of aromatic compounds by olefins. For example, Neuber, et al. in U.S. Patent No. 5,396,012 disclose a process for the production of monoisopropylnaphthalene by the alkylation of naphthalene using a faujasite type zeolite Y catalyst in which the cations of the zeolite have been replaced by alkaline earth metal ions.

## Summary of the Invention

It is an object of this invention to provide improvements in the process for the production of butenylated biphenyl so that higher yields of the desired sec-butylbiphenyl and di-sec-butylbiphenyl products are obtained.

It is also an object of this invention to provide improvements in the described process which reduce the types and amounts of undesirable side products which have an objectionable odor so that the butenylated biphenyl has a lower odor than is currently obtainable.

To obtain these and other related objects, in one aspect the invention is directed to a process for production of sec-butylbiphenyl and/or di-sec-butylbiphenyl comprising contacting butene and biphenyl in the presence of a catalyst complex under alkylation conditions effective to form sec-butylbiphenyl and/or di-sec-butylbiphenyl as an alkylation product of the butene and said biphenyl. The catalyst complex comprises a Lewis acid, an aromatic hydrocarbon and a hydrogen halide or alkyl halide, wherein more than about 50 mole % of the alkyl group substituents on said aromatic hydrocarbon are methyl groups. If desired or necessary, the alkylation product can be held at the reaction temperature or further Lewis acid catalyst complex can added to transalkylate the alkylation product. The alkylation process may alternatively be carried out in the presence of a zeolite catalyst selected from one or more of Y zeolites, USY zeolites, mordenite zeolites, ZSM-12 zeolites, and FCC catalysts.

In another aspect, the invention is directed to a process for preparing a sec-butylbiphenyl and/or di-sec-butylbiphenyl product comprising contacting a feedstock containing two or more of biphenyl, sec-butylbiphenyl, di-sec-butylbiphenyl and tri-sec-butylbiphenyl with a Lewis acid catalyst complex under transalkylation conditions effective to transfer sec-butyl groups between said two or more of said biphenyl, sec-butylbiphenyl, di-sec-butylbiphenyl and tri-sec-butylbiphenyl and form said sec-butylbiphenyl and/or di-sec-butylbiphenyl product. Again, alternatively, the zeolite catalyst can be used in place of the Lewis acid catalyst complex.

In a further aspect, the invention is directed to products made from the foregoing processes, such products including microcapsules containing solubilized dye for use in carbonless paper systems.

## Brief Description of the Drawings

Fig. 1 is a graph showing alkylation curves for alkylation compositions at various mole ratios of butene:biphenyl for an alkylation occurring at 120°C, using the Lewis acid catalyst complex;

Fig. 2 is a graph similar to Fig. 1 for alkylation occurring at 80°C, using the Lewis acid catalyst complex;

Fig. 3 is a graph similar to Fig. 1 and Fig. 2 for alkylation using a zeolite catalyst; and

Fig. 4 is a graph showing the statistical analysis of comparative testing of the odor of products of the invention.

## Detailed Description of the Invention

In accordance with the present invention, it has been found that alkylation of biphenyl by butene, when carried out under specific conditions, produces a product, which, after distillation, has a lower odor than butenylated biphenyl produced under previously disclosed conditions. Alkylation can occur using either Lewis acid catalysis or solid acid catalysis. The process of the present invention produces higher yields of sec-butylbiphenyl and di-sec-butylbiphenyl than the process of the prior art. It has also been discovered that the process of the present invention produces sec-butylbiphenyl and di-sec-butylbiphenyl products which contain fewer minor impurities.

## Lewis Acid Catalysis

Complexes of Lewis acid catalysts such as aluminum chloride, aluminum bromide, tin chloride, titanium chloride, boron trifluoride, and the like are useful in the present invention for the production of butenylated biphenyl by alkylation of biphenyl by butene include. These complexes, sometimes called "red oils" or "catalyst sludges", are well known in the art and are formed when the Lewis Acid is suspended in aromatic hydrocarbons while a hydrogen halide or alkyl halide is added. The mole ratio of Lewis acid:aromatic hydrocarbon:hydrogen or alkyl halide is typically 2:1:0.5. A catalyst complex is formed which acts as a "super acid". These catalyst complexes are one preferred catalyst in the inventive process. The aluminum chloride aromatic hydrocarbon methyl chloride catalyst complex is most preferred.

The aromatic hydrocarbon in the preferred catalyst complex can be an alkyl- or polyalkylbenzene such as cumene (isopropylbenzene), xylenes (pure isomers or mixtures), trimethylbenzenes, tetramethylbenzenes, mixtures of ethylbenzenes, diethylbenzenes, triethylbenzenes, dimethylethylbenzenes, diisopropylbenzenes, triisopropylbenzenes, or mixtures of some, most, or all of the above, along with other similar aromatic and alkylaromatic hydrocarbons, including those containing more than 1 aromatic ring, such as biphenyl, naphthalene, or the like.

It has been found that aromatic hydrocarbons used in the catalyst complex, in which a majority of the contained alkyl groups are methyl groups, led to an alkylate which is easier to distill and which provides a product which has a lower odor. Compared to ethyl alkyl groups and propyl (or isopropyl) alkyl groups, methyl alkyl groups are less likely to transalkylate onto the biphenyl molecules during the alkylation. Transalkylation of the alkyl groups onto the biphenyl molecules can lead to impurities which lower the yield and generate products with higher odors. Propyl (or isopropyl) alkylbiphenyls are difficult to separate from the butylbiphenyl components and have a more intense odor than the butylbiphenyl components. Ethylbiphenyls are not as difficult to separate from butylbiphenyl as the propyl (or isopropyl) biphenyls, but they none-the-less can cause odor and purity problems.

For these aforementioned reasons, it is preferred that the aromatic hydrocarbon used in the catalyst complex have ≥ 50 mole % methyl groups, based on total moles of alkyl groups in the aromatic hydrocarbon. It is more preferred that the aromatic hydrocarbon used in the catalyst complex have ≥ 70 mole % methyl groups, based on the total moles of alkyl groups in the aromatic hydrocarbon. It is most preferred that the aromatic hydrocarbon have ≥ 80 mole % methyl groups, based on the total moles of alkyl groups in the aromatic hydrocarbon.

The amount of catalyst complex can vary widely, from about 0.01 wt % of the parent Lewis acid (based on the weight of the biphenyl) to about 10 wt % of the parent Lewis acid, with 0.1 to 5 wt % of the parent Lewis acid more preferred, with about 0.5 to 2 wt% of the parent Lewis acid being most preferred.

The temperature of the alkylation reaction using the Lewis acid can vary from about 0°C to 115°C, with 25°C to 100°C being more preferred and 40°C to 90°C being most preferred. Below about 70°C to about 75°C special steps need to be taken to keep the biphenyl from freezing out of the reaction, at least during the early part of the alkylation. Pure biphenyl has a freezing point of 69-72°C; the 95% biphenyl used in most of the experimental has a freezing point of about 66°C. Introduction of the catalyst complex into the molten biphenyl lowers its freezing point to 60°C to 50°C or lower depending on the catalyst complex and how much is used.

Addition of further diluents or reactants allows the alkylation to occur at an even colder temperature. One reactant which is being added continuously is the olefin, in this case the butene. As the butene is added, the biphenyl concentration becomes lower as sec-butylbiphenyl and di-sec-butylbiphenyl are being formed. This allows the temperature of the reaction mixture to be lowered as the alkylation proceeds.

Other reactants which can be added to lower the freezing point of the mixture are sec-butylbiphenyl, di-sec-butylbiphenyl, tri-sec-butylbiphenyl, or mixtures of two or more of these by themselves or with biphenyl, such as would be obtained from a previous alkylation. In this manner, the alkylation can begin at 70°C or lower and the reaction temperature can be lowered gradually to 40°C or lower. If solid biphenyl is observed precipitating out on the walls of the reaction vessel, the temperature of the mixture can be raised slightly to dissolve the solids or the alkylation can be held at that temperature as eventually the solids will dissolve.

Non-reactive diluents such as solvents can be added to the reaction mixture to allow the alkylation to be carried out a low temperatures. Solvents such as methylene chloride (dichloromethane), ethylene dichloride (1,2-dichloroethane), chlorobenzene, hexane, cyclohexane, heptane, octane, and the like can be used, along with mixtures of two or more of these solvents. Methylene chloride or ethylene dichloride are the preferred solvents.

If a reactive diluent is added, it is added in the amount of 1 wt % to about 20 wt % based on the weight of the biphenyl. The amount of Lewis acid catalyst complex used is then based on the combination of the weight of the reactive diluent + the weight of the biphenyl. The Lewis acid catalyst complex is used in the same proportions as stated above.

If a non-reactive diluent is added, it is added in the amount of 1 volume % to about 100 volume %, based on the volume of the biphenyl (or the volume of the biphenyl + reactive diluent), with about 10 volume % to about 50 volume % preferred.

The color of the reaction mixture at the lower temperatures of the present invention is deep dark red or purple rather than the black color that is typical when the reaction is carried out at 120°C or higher. The quenched alkylate is usually colorless to light yellow rather than the dark yellow to black which is obtained when the reaction is carried out at 120°C temperature or higher.

The olefin used is butene, which can be in the form of 1- or 2-butene (either *cis* or *trans*-2-butene) or mixtures of two or more of these butene isomers. Either pure butene can be used or a mixture of butene with non-reactive diluents such as butane, nitrogen, or the like, such as is obtained from a petroleum refinery stream. Commercially available butene sometimes has small amounts of butadiene, isobutylene, and the like. These do not interfere with the inventive process as long as they do not represent too high a proportion of the mixture. Isobutylene produces t-butylbiphenyl in the product. Butadiene produces heavy by-products.

The butene is introduced as a gas below the surface of the reaction mixture using a glass tube, metal cannula, teflon coated pipe, or a similar gas/liquid contact method, as is known in the art.

The pressure of the preferred reaction process can vary anywhere from sub ambient pressure to 100 lbs per square inch gauge (psig), with 0 to 20 psig being more preferred, with 0 to 10 psig being most preferred. If non-reactive diluents are present in the butene, provisions may be made for venting excess non-reactive diluent, and the reaction is usually run under a moderate amount of pressure, about 10-20 psig.

The amount of butene added is enough to provide from about 0.2 mole equivalent, based on biphenyl, to about 2.6 mole equivalents. The preferred amount of butene added is from about 0.4 to about 1.0 mole equivalents. The higher range of about 1.0 to about 2.6 mole equivalents is used when a heavier product, composed of mostly the di-sec-butylbiphenyl, is being produced in addition to the one containing mostly sec-butylbiphenyl herein described. If the only product being produced is the one containing mostly sec-butylbiphenyl, then the lower range of about 0.4 to about 1.0 mole equivalents butene is preferred.

The time of the reaction can vary from about 0.5 hr to about 24 hr. Preferred times are whatever is necessary to allow the butene to be added while still controlling the temperature of the process. Preferred times are from about 1 hr to about 12 hr, with about 2 hr to about 8 hr being more preferred.

Once the addition of the butene is completed, the reaction can be quenched, or the reaction can be allowed to reach "thermodynamic equilibrium". If the reaction is quenched without any further treatment or hold time, it is generally said to be a kinetic product rather than a thermodynamic product. A kinetic product is one in which the various isomers are the ones which are formed first. A thermodynamic product is one in which the various isomers are the ones which are more stable. A thermodynamic product is preferred.

There are generally two methods which are used to insure that the reaction has reached thermodynamic equilibrium. In one method, the reaction mixture is held at a certain temperature until it has been deemed to have completed. The temperature at which the reaction mixture is held is generally the same temperature at which the butene has been added. This hold time allows the reaction mixture to reach a thermodynamic equilibrium through transalkylation of the alkyl groups, a process well known in the art. Hold times can vary from 0.5 hr to 12 hr, with 1 hr to 3 hr being more preferred.

Another method which is used to drive the reaction mixture toward thermodynamic equilibrium is to add another portion of catalyst complex. Generally, the amount of catalyst complex added is about the same as was added for the initial alkylation (although more or less can be used, depending on the activity of the catalyst), and the temperature is about the same as for the initial alkylation. Transalkylation times vary from about 0.25 hr to about 12 hr, with 1.0 hr to about 3.0 hr being more preferred.

During the transalkylation, the sec-butyl substituents are rearranged until thermodynamic equilibrium is reached. In general, this may involve substitution of a sec-butyl group on a biphenyl and removal of a sec-butyl group from di-sec-butylbiphenyl to form sec-butylbiphenyl.

Once the reaction is complete, it is quenched using methods well known in the art, such as addition of aqueous sodium hydroxide (caustic soda). The quenched alkylate is separated from the aqueous layer, sometimes washed again with water, then it is distilled using methods well known in the art. The final product is that which contains about 100% to about 0% sec-butylbiphenyl with about 0% to about 100% di-sec-butylbiphenyl. A preferred composition for use as a dye solvent for use in carbonless paper systems contains about from 50% to 100% sec-butylbiphenyl range with about 45% to about 0% di-sec-butylbiphenyl range. Other minor components are present in amounts ranging from about 10% to about 0%. A preferred composition contains about from 80% to 84% sec-butylbiphenyl with about 10% to 14% di-sec-butylbiphenyl. This preferred composition for use as a dye solvent contains from about 10% to about 0% isobutylbiphenyl. All percentages are by weight.

Analysis of this product is accomplished by the use of gas chromatography. Analysis techniques available at the time of the invention disclosed in U.S. Patent No. 4,287,074 were generally not capable of separating the sec-butylbiphenyl isomers from the isobutylbiphenyl isomers. Capillary gas chromatography, such as is available in the present day, is capable of separating the 3-sec-butylbiphenyl isomer from the 3-isobutylbiphenyl isomer and the 4-sec-butylbiphenyl isomer from the 4-isobutylbiphenyl isomer. The use of capillary columns such as the 60 meter DB®-1 column and the 100 m Petrocol® column enables the separation of these isomers. Temperature programs such as an initial temperature of 150°C, ramping 3°C/min to 215°C, ramping 30°C per minute to 320°C, with a hold at 320°C, allow the separation of the isomers on a DB®-1 column. A similar program is used on the 100 m Petrocol® column, with a lower initial temperature of 125°C with a 2°C/min ramp to 215°C. The di-sec-butylbiphenyl isomers are also separated from the diisobutylbiphenyl and the sec-butyl-isobutylbiphenyl isomers under these conditions.

Authentic 4-isobutylbiphenyl was produced by acylation of biphenyl by isobutyryl chloride followed by reduction of the ketone group, a process known in the art. The identity of the 3-isobutylbiphenyl was determined by a process of elimination, and was based on its gas chromatographic retention time compared to the 3-sec-butylbiphenyl.

Without being limited by theory, it appears that the reduced odor of the products produced in the inventive process is due to the lower amount of isobutylbiphenyl isomers present in the product. Thus, the prior art process produces a product which contains greater than about 12% 3-isobutylbiphenyl + 4-isobutylbiphenyl. This was not apparent at the time of the disclosure of the prior art due to the ineffectiveness of the analytical technique used. The product produced using the inventive process has an isobutylbiphenyl content of 10% or less, preferably 6% or less, more preferably 3% or less, and it has a discernibly lower, less intense odor than the product produced using the prior art process described in U.S. Patent No. 4,287,074.

Another reason for the reduced odor of the product from the inventive process is that fewer impurities are produced at the lower temperature of the inventive process compared to the conditions of the prior art. When the higher temperatures of the prior art are used, fragmentation of the alkyl groups occurs, producing $C_3$, $C_2$, and $C_1$ alkyl groups. The lower temperatures of the inventive process avoid such fragmentation.

It has been found that the choice of aromatic hydrocarbon used in the catalyst complex is important. When, for instance, cumene is used in the catalyst complex, a small amount of isopropylbiphenyl impurity, about 2% of the alkylate, is produced due to transalkylation of the isopropyl group from the cumene to the biphenyl. The isopropylbiphenyl impurity is difficult to purify away from the sec-butylbiphenyl. The isopropylbiphenyl has a high odor and is detrimental to the odor of the product. Therefore it is preferable to use aromatic hydrocarbons in the catalyst complex which do not have propyl or isopropyl groups. However, it has been determined that even the use of cumene in the catalyst complex for the inventive process will still lead to a purified product which has a lower odor than the prior art product.

Aromatic hydrocarbons containing ethyl alkyl groups are also not preferred for use in the catalyst complex since the ethyl alkyl groups will transalkylate onto the biphenyl and cause impurity and odor problems. Ethylbiphenyls are easier to purify away from the sec-butylbiphenyl than are isopropylbiphenyl, so the presence or absence of ethyl groups in the aromatic hydrocarbon is not as important as is the absence of propyl and isopropyl groups. However, the highest quality of product will be produced from the inventive process when the aromatic hydrocarbon used in the catalyst complex does not contain appreciable amounts of ethyl alkyl groups.

For these aforementioned reasons, it is preferred that the aromatic hydrocarbon used in the catalyst complex have ≥ 50 mole % methyl groups, based on total moles of alkyl groups in the aromatic hydrocarbon. It is more preferred that the aromatic hydrocarbon used in the catalyst complex have ≥ 70 mole % methyl groups, based on the total moles of alkyl groups in the aromatic hydrocarbon. It is most preferred that the aromatic hydrocarbon have ≥ 80 mole % methyl groups, based on the total moles of alkyl groups in the aromatic hydrocarbon.

Since odor is a subjective quality, it is plausible that as-of-yet unknown impurities, possibly in very small quantities, could be causing the differential odors between the butenylated biphenyl of the prior art and the butenylated biphenyl of the present invention. Therefore, the scope of our invention is not to be limited due to the theory that the odor difference is caused by the lower concentration or absence of the isobutylbiphenyl isomers. Those skilled in the art will appreciate that the overall claim of the lower odor produced using the inventive process will still hold.

## Zeolite Catalysis

Zeolites are well known in the art; they are solid acid catalysts composed of mixtures of silica and alumina which have caged structures. The pores of the caged structures allow molecules to enter the catalyst where reactions take place. Typical zeolites are faujasites, of which Y zeolites are a member, mordenite zeolites, and pentasil zeolites such as ZSM-5, ZSM-12, and so on. These zeolites can exist in a form, such as a sodium form, in which they are relatively inactive for the inventive process. When the zeolites are "acid exchanged" by methods known in the art they become active for the inventive process.

The acid form of the Y zeolite, for instance, is called the HY form. The HY zeolite is also called the "ultra stable Y", or USY, because removal of the sodium cations produces a more stable structure. USY zeolites are the most heavily industrially used catalysts for hydrocarbon conversions. For example, USY catalysts are used in fluid catalytic cracking units (FCC units) to produce short chain hydrocarbons from the long chain hydrocarbons found in crude oil. FCC catalysts are usually not pure Y zeolites, but are mixtures of Y zeolite and a binder and matrix. For instance, it is known in the art that Octacat® FCC catalyst is a mixture of about 15 to 60 wt % USY, with the remainder being silica-alumina sol binder and kaolin matrix. The binder serves to increase the strength of the catalyst particle. The matrix is the material throughout which the zeolite is spread. It may or may not have any activity of its own. Since the exact composition of FCC catalysts is proprietary to each company making the catalysts, the present inventors do not know the exact composition of the FCC catalysts of the present application. Millions of lbs of USY catalysts are used in FCC units every year.

These zeolite catalysts can be used in the inventive process to produce a product which has a lower odor than the product produced using the prior art process.

The zeolites used in the inventive process include Y zeolites, USY zeolites, mordenite zeolites, ZSM-12 zeolites, FCC catalysts, and spent FCC catalysts. Spent FCC catalysts have been discharged from FCC units because they are no longer highly active in the FCC unit and/or their particle size has been degraded in the FCC unit so that processing them is difficult. However, spent FCC catalysts often retain enough of their activity so that they can be used in other FCC units or in other processes such as the inventive process. The Y zeolites preferably have a pore size of about 0.7 to 0.9 nm while the mordenite zeolites preferably have a pore size of about 0.6 to 0.9 nm.

A drying stage is often necessary to remove water from the zeolites before they have substantial activity in the inventive process. This is known in the prior art. Drying of the zeolites in an oven or furnace at 300°C to 600°C, preferably from 350°C to 550°C, more preferably from 450°C to 550°C, produces catalysts which might still contain water but which are quite active in the inventive process.

There have also been claims in the prior art that the presence of ammonium or other large diameter cations in the zeolites is beneficial to the alkylation process. This has been confirmed in the inventive process by not completely drying acid-exchanged Y zeolite, thus allowing some ammonia to remain. However, it is not necessary to have such a catalyst containing ammonium ion for the successful carrying out of the inventive process.

The preferred catalyst in the inventive process is a Y zeolite, either fresh or in the form of spent FCC catalyst. The more preferred catalyst is a USY, either fresh or in the form of a spent FCC catalyst. The most preferred catalyst is an FCC catalyst, either fresh or spent. Catalysts such as the Y zeolites from Conteka (now PQ): CBV 760, CBV 720, and CBV 500; the Y zeolites from UOP: Y-84, LZY-15; FCC catalysts such as Octacat®, Octacat +®, Super Octacat®, or Orion® from Grace-Davison; FCC catalysts such as Horizon®, KOB®, Horizon/Vision®, or Eclipse® from Akzo; FCC catalysts such as Reduxion®, Precision®, or Vektor® from Engelhard; or spent FCC catalysts such as any of those FCC catalysts previously named which have been obtained in their spent form from FCC units. One source of the spent FCC catalysts is the Rocky Mountain Salvage Company.

Preferred temperatures for the zeolite catalyzed reaction are from about 50°C to about 300°C ; the more preferred range is from about 120°C to about 260°C; the most preferred range is from about 140°C to about 200°C.

The olefin used for the aklylation of biphenyl is butene, which can be in the form of 1- or 2-butene (either *cis* or *trans*-2-butene) or mixtures of two or more butene isomers. Either pure butene can be used or a mixture of butene with non-reactive diluents such as butane, nitrogen, or the like, such as is obtained from a petroleum refinery stream. Commercially available butene sometimes has small amounts of butadiene, isobutylene, and the like. These do not interfere with the inventive process as long as they do not represent too high a proportion of the mixture.

The butene is introduced as a gas below the surface of the reaction mixture using a glass tube, metal cannula, or liquid/gas contacting method, known in the art.

The pressure of the preferred reaction process can vary anywhere from the ambient pressure to about 100 lbs per square inch gauge (psig), with 0 to 20 psig being more preferred, with 0 to 10 psig being most preferred. If non-reactive diluents are present in the butene, means for gassing them off must be provided, and the reaction is usually run under a moderate amount of pressure, about 10-20 psig.

The amount of butene added is enough to provide from about 0.2 mole equivalent, based on biphenyl, to about 2.6 mole equivalents. The preferred amount of butene added is from about 0.4 to about 1.0 mole equivalents. The higher range of about 1.0 to about 2.6 mole equivalents is used when another product, composed of mostly the di-sec-butylbiphenyl, is being produced in addition to the CPS containing mostly sec-butylbiphenyl herein described. If the only product being produced is the one containing mostly sec-butylbiphenyl, then the lower range of about 0.4 to about 1.0 mole equivalents butene is preferred.

The time of the reaction can vary from about 0.1 hr to about 24 hr. Preferred times are whatever is necessary to allow the butene to be added while still controlling the temperature of the process. Preferred times are from about 1 hr to about 12 hr, with about 2 hr to about 8 hr being more preferred.

The preferred zeolite catalyst dose is from about 1 wt % based on biphenyl to about 20 wt % based on biphenyl. The more preferred zeolite catalyst dose is from about 5 wt % to about 15 wt % based on biphenyl. The most preferred zeolite catalyst dose is from about 7.5 wt % to about 12.5 wt % based on biphenyl.

In the process on the invention, it is necessary to first mix the molten biphenyl with the zeolite catalyst. Introduction of the butene before the catalyst and biphenyl have been fully mixed is less preferred.

There is no hold time nor transalkylation time or catalyst necessary for the process catalyzed by a zeolite. Once the butene has been added, the catalyst is filtered from the reaction mixture using any means as is known in the art. The filtered alkylate can be directly distilled, without the necessity for water quenching or washing.

It is within the scope of this invention that instead of using batch reactions to carry out the alkylation, a semi-continuous or continuous process could be used to produce the butenylated biphenyl alkylate. In such cases the operating conditions would essentially be the same except that the feedstocks would be flowing through a catalyst packed reactor continuously, or, in the case of the Lewis acid catalyst complex catalyzed reaction, the feedstocks would be flowing into a stirred tank to which the catalyst complex was added continuously, and the product would flow out an outlet of the tank. For the zeolite catalyzed continuous or semi-continuous reaction, such continuous or semi-continuous reactors could be in the form of a fixed bed, such as with a tube packed with a stationary portion of catalyst particles, or the continuous or semi-continuous reactor could be in the form of a tank containing a slurry bed of catalyst or the like, or the continuous or semi-continuous reactor could be in the form of a fluidized bed reactor.

In the continuous or semi-continuous alkylation processes, the reaction conditions such as the temperature, pressure, and average time in the reaction zone, would be the same as in the batch processes previously described.

The product alkylate from the zeolite cataylzed process, whether produced from a batch or continuous reaction, is distilled using methods well known in the art. The final product for use as a carbonless paper solvent is that which contains about 50% to about 100% sec-butylbiphenyl with about 45% to about 0% di-sec-butylbiphenyl. A preferred composition contains about from 80% to 84% sec-butylbiphenyl with about 10% to about 14% di-sec-butylbiphenyl. Other minor components are present in amounts ranging from about 10% to about 0%. This preferred composition for use as a dye solvent contains from about 10% to about 0% isobutylbiphenyl. All percentages are by weight.

As is the case with the Lewis acid catalyzed process, the final product from the zeolite catalyzed process has a reduced amount of isobutylbiphenyl as compared to the product from the previously disclosed process of the existing art. It has a lower odor but retains the other positive physical properties of the butenylated biphenyl products.

## Production of Butenylated Biphenyl Using Transalkylation

Another process of the present invention which can be used to produce butenylated biphenyl is to carry out a transalkylation of certain types of feedstocks rather than an alkylation. In manufacturing processes such as the one described in the inventive process, one often obtains fractions from distillations which are not appropriately used directly in the product but are none-the-less valuable due to their sec-butylbiphenyl content. These fractions can be used as feedstocks in transalkylation processes.

For instance, in the practice of the present invention, the unreacted excess biphenyl is distilled away from the alkylate. Since the separation by distillation is not perfect, the distilled biphenyl often contains portions of the desired sec-butylbiphenyl. In addition, when all of the butenylated biphenyl product is distilled overhead, there often remains a heavier material, a mixture of di-sec-butylbiphenyl and possibly tri-sec-butylbiphenyl, which can also be distilled overhead or, as is sometimes the case, it can be drained out of the still as "still bottoms". This heavier material has value.

Mixtures of the recovered biphenyl, sec-butylbiphenyl, and di-sec-butylbiphenyl (and sometimes tri-sec-butylbiphenyl) can be transalkylated by adding catalyst at certain reaction temperatures. In the transalkylation, a new equilibrium is attained in which the sec-butyl groups are transferred between molecules until thermodynamic equilibrium is reached. In such a case, the "average alkyl groups" (average number of sec-butyl groups per biphenyl molecule) will be the same or nearly the same as the average alkyl groups of an alkylate which contains the same mole ratio of butene:biphenyl.

To explain further; a transalkylate which contains a mole ratio butene:biphenyl of 1.00 will have approximately the same composition as an alkylate which contains a mole ratio butene:biphenyl of 1.00, as long as they have both reached a thermodynamic equilibrium. It does not matter if, at the beginning of the transalkylation, all of the butyl groups in a mixture of biphenyl and di-sec-butylbiphenyl are contained in the di-sec-butylbiphenyl component of the mixture. At the end of the reaction, the butyl groups will have spread throughout the mixture and a mixture of biphenyl, sec-butylbiphenyl, di-sec-butylbiphenyl and possibly tri-sec-butylbiphenyl will be obtained.

The average alkyl group (AAG) content of the transalkylation feedstock is calculated using equation 1:

$$AAG = \left[\frac{\dfrac{GC\%SBBP}{FWSBBP} + 2 \cdot \dfrac{GC\%DSBBP}{FWDSBBP} + 3 \cdot \dfrac{GC\%TSBBP}{FWTSBBP}}{\dfrac{GC\%Biphenyl}{FWBiphenyl} + \dfrac{GC\%SBBP}{FWSBBP} + \dfrac{GC\%DSBBP}{FWDSBBP} + \dfrac{GC\%TSBBP}{FWTSBBP}}\right] \quad (1)$$

where

AAG = average alkyl group
GC % SBBP = GC area % sec-butylbiphenyl region
GC % DSBBP = GC area % di-sec-butylbiphenyl region
GC % TSBBP = GC area % tri-sec-butylbiphenyl region
FW SBBP = formula weight sec-butylbiphenyl
FW DSBBP = formula weight di-sec-butylbiphenyl
FW TSBBP = formula weight tri-sec-butylbiphenyl
GC % Biphenyl = GC area % biphenyl
FW Biphenyl = formula weight biphenyl
(In equation 1, it is assumed the GC area % corresponds to weight %.)

The feedstock for such transalkylations can be mixtures of products from Lewis acid catalyzed reactions, from zeolite catalyzed reactions, or from clay or other solid acid catalyzed reactions, alone or from two or more of these reactions. One of the key factors in determining whether or not the feedstock is a good feedstock for transalkylation is how much isobutylbiphenyl it contains, with no or low amounts of isobutylpiphenyl being preferred. In general, those feedstocks which are used to produce low odor butenylated biphenyl (LOBBP) are good feedstocks for the transalkylation.

The preferred Lewis acid catalysts for production of transalkylation feedstocks are the same as those detailed above. The preferred reaction conditions for production of these transalkylation feedstocks when using Lewis acid catalysts are the same as those detailed above. The important factor is that the isobutylbiphenyl content is low in the feedstock.

The preferred zeolite catalysts for production of transalkylation feedstocks are the same as those detailed above, as are the reaction conditions.

The preferred clay catalysts for production of these transalkylation feedstocks include acid treated clays such as Harshaw-Filtrol® 13LM or Grade 24. Other clays with similar properties can be used (Harshaw-Filtrol® 13LM is no longer made and Ingelhard® Grade F-20XLM).

Preferred temperatures for the clay catalyzed alkylations are from about 80°C to about 300°C, with a more preferred range of from about 100°C to about 250°C, with a most preferred range of about from 120°C to 200°C.

The amount of clay catalyst used in the reaction is from about 1 wt % based on the biphenyl feedstock to about 20 wt % based on the biphenyl feedstock, with a more preferred range of about 5 wt % to about 15 wt %, with a most preferred range of about 7 wt % to about 12 wt %.

The clay can be in the form of a powder or in granules, pellets, spheroids, etc.

In the production of the transalkylation feedstock by clay catalysis, the olefin used is butene, which can be in the form of 1- or 2-butene (either *cis* or *trans*-2-butene) or mixtures of two or more of these butene isomers. Either pure butene can be used or a mixture of butene with non-reactive diluents such as butane, nitrogen, or the like, such as is obtained from a petroleum refinery stream. Commercially available butene sometimes has small amounts of butadiene, isobutylene, and the like. These do not interfere with the inventive process as long as they do not represent too high a proportion of the mixture.

The butene is introduced as a gas below the surface of the reaction mixture using a glass tube, metal cannula, teflon coated pipe, or a similar gas/liquid contact method, many of which are known in the art.

The pressure of the preferred reaction process can vary anywhere from sub ambient pressure to 100 lbs per square inch gauge (psig), with 0 to 20 psig being more preferred, with 0 to 10 psig being most preferred. If non-reactive diluents are present in the butene, provisions may be made for venting excess non-reactive diluent, and the reaction is usually run under a moderate amount of pressure, about 10-20 psig.

The amount of butene added is enough to provide from about 0.2 mole equivalent, based on biphenyl, to about 2.6 mole equivalents. The preferred amount of butene added is from about 0.4 to about 1.0 mole equivalents.

It has been found that alkylation of biphenyl by butene catalyzed by these clay catalysts produces very little isobutylbiphenyl, often less than 10% total isobutylbiphenyl, preferably less than 5% isobutylbiphenyl, more preferably less than 3% isobutylbiphenyl, most preferably less than 1% isobutylbiphenyl.

The ratios of the various sec-butylbiphenyl and di-sec-butylbiphenyl isomers in the clay catalyzed reaction are inappropriate for use of the alkylate directly in production of low odor butenylated biphenyl. Generally, there is too much ortho-substituted sec-butylbipheny and di-sec-butylbiphenyl, and the yield of low odor butenylated biphenyl product will be lower than normal. However, transalkylation of all or part of the alkylate from the clay-catalyzed reactions will produce a transalkylate which can be used to make low odor butenylated biphenyl product.

The preferred Lewis acid catalysts for the transalkylation include aluminum chloride, aluminum bromide, tin chloride, titanium chloride, boron trifluoride, and the like. Complexes of Lewis acids are active in the inventive process. These complexes, sometimes called "red oils" or "catalyst sludges", are well known in the art and are formed when aluminum chloride, aluminum bromide or the like are suspended in aromatic hydrocarbons while a hydrogen halide or alkyl halide is added. The mole ratio of Lewis acid:aromatic hydrocarbon hydrogen or alkyl halide is typically 2:1:0.5. A catalyst complex is formed which acts as a "super acid". These catalyst complexes are one preferred catalyst in the inventive process. The aluminum chloride aromatic hydrocarbon methyl chloride catalyst complex is most preferred.

It is preferred that the aromatic hydrocarbon used in the catalyst complex have ≥50 mole % methyl groups, based on total moles of alkyl groups in the aromatic hydrocarbon. It is more preferred that the aromatic hydrocarbon used in the catalyst complex have ≥ 70 mole % methyl groups, based on the total moles of alkyl groups in the aromatic hydrocarbon. It is most preferred that the aromatic hydrocarbon have ≥ 80 mole % methyl groups, based on the total moles of alkyl groups in the aromatic hydrocarbon.

The average alkyl group (AAG) content of the transalkylation mixture before addition of the catalyst can vary from about 0.2 to 2.6, preferably from about 0.4 to 1.5, most preferably from about 0.6 to 1.2 average alkyl groups per biphenyl molecule.

The amount of catalyst complex can vary widely, from about 0.01 wt % of the parent Lewis acid (based on the weight of the total feedstock) to about 10 wt % of the parent Lewis acid, with 0.1 to 5 wt % of the parent Lewis acid more preferred, with about 0.5 to 2 wt % of the parent Lewis acid being most preferred.

The temperature of the transalkylation reaction using the Lewis acid can vary from about 0°C to 90°C, with 25°C to 80°C being more preferred and 50°C to 70°C being most preferred.

The pressure of the preferred transalkylation reaction process can vary anywhere from sub ambient pressure to 100 lbs per square inch gauge (psig), with 0 to 20 psig being more preferred, with 0 to 10 psig being most preferred.

The time of the transalkylation reaction can vary from about 0.25 hr to about 12 hr. Preferred times are whatever is necessary to allow the transalkylation to come to equilibrium, or from about 0.5 hr to about 12 hr, with about 1 hr to about 3 hr being more preferred.

Once the transalkylation is completed the reaction mixture is quenched and distilled as described for the alkylate process. Low odor butenylated biphenyl product made from the transalkylate has a low odor and retains the other positive properties of butenylated biphenyl made via the prior art.

It is also possible to carry out the transalkylation using zeolite catalyst. The preferred catalyst in the transalkylation process is a Y zeolite, either fresh or in the form of spent FCC catalyst. The more preferred catalyst is a USY, either fresh or in the form of a spent FCC catalyst. The most preferred catalyst is an FCC catalyst, either fresh or spent. Catalysts such as the Y zeolites from Conteka (now PQ): CBV 760, CBV 720, and CBV 500; the Y zeolites from UOP: Y-84, LZY-15; FCC catalysts such as Octacat®, Octacat +®, Super Octacat®, or Orion® from Grace-Davison; FCC catalysts such as Horizon®, KOB®, Horizon/Vision®, or Eclipse® from Akzo; FCC catalysts such as Reduxion®, Precision®, or Vektor® from Engelhard; or spent FCC catalysts such as any of those FCC catalysts previously named which have been obtained in their spent form from FCC units. One source of the spent FCC catalysts is the Rocky Mountain Salvage Company.

Preferred temperatures for the zeolite catalyzed transalkylation are from about 50°C to about 300°C; the more preferred range is from about 120°C to about 260°C; the most preferred range is from about 140°C to about 200°C.

The time of the transalkylation can vary from about 0.1 hr to about 24 hr, with about 1 hr to about 12 hr being more preferred, and from about 3 hr to about 8 hr being most preferred.

The average alkyl group (AAG) content of the transalkylation mixture before addition of the catalyst can vary from about 0.2 to 2.6, preferably from about 0.4 to 1.5, most preferably from about 0.6 to 1.2 average alkyl groups per biphenyl molecule.

The preferred zeolite catalyst dose is from about 1 wt % based on total feedstock to about 20 wt % based on total feedstock. The more preferred zeolite catalyst dose is from about 5 wt % to about 15 wt % based on total feedstock. The most preferred zeolite catalyst dose is from about 7.5 wt % to about 12.5 wt % based on total feedstock.

Once the transalkylation is complete, the catalyst is filtered from the reaction mixture using any means as is known in the art. The filtered transalkylate can be directly distilled, without the necessity for water quenching or washing.

It is within the scope of this invention that instead of using batch reactions to carry out the transalkylation, one could use a semi-continuous or continuous process to produce the butenylated biphenyl transalkylate. In such cases the operating conditions would essentially be the same except that the feedstocks would be flowing through a catalyst packed reactor continuously, or through a tank to which the Lewis acid catalyst complex was being continuously added. In the case of zeolite catalyzed transalkylation, such continuous reactors could be in the form of a fixed bed, such as with a tube packed with a stationary portion of catalyst particles, or the continuous reactor could be in the form of a tank containing a slurry bed of catalyst or the like, or the continuous reactor could be in the form of a fluidized bed reactor.

In the continuous transalkylation processes, the reaction conditions as far as temperature, pressure, average time in the reaction zone, etc. are concerned would be the same as in the batch processes previously described.

The product alkylate, whether produced from a batch or continuous transalkylation reaction, is distilled using methods well known in the art. The final product for use as a carbonless paper solvent is that which contains about 50% to about 100% sec-butylbiphenyl range components with about 45% to about 0% di-sec-butylbiphenyl range components. A more preferred composition contains about from 80% to 84% sec-butylbiphenyl range components with about 10% to about 14% di-sec-butylbiphenyl range components. Other minor components are present in amounts ranging from about 5% to about 10%. This preferred composition for use as a dye solvent contains from about 10% to about 0% isobutylbiphenyl. All percentages are by weight.

As is the case with the Lewis acid or zeolite catalyzed alkylation process, the final product from the Lewis acid or zeolite catalyzed transalkylation process has a reduced amount of isobutylbiphenyl as compared to the product from the previously disclosed process of the existing art. It has a lower odor but retains the other positive physical properties of the butenylated biphenyl products.

## Experimentals

Products produced at the conditions of the inventive process are designated as LOBBP when they are intended for use as a carbonless paper solvent. A number is appended in parentheses which indicates the temperature in °C at which the LOBBP was produced. Using this nomenclature, LOBBP(60) for example was produced at 60°C using the inventive process.

Sure Sol®-125 (Koch Chemical Co.) is an aromatic solvent containing about 46% by weight C9's and C10's which only contain methyl alkyl groups and 27% by weight C9's and C10's which contain mixtures of one methyl alkyl group and one ethyl alkyl group or two methyl alkyl groups and one ethyl alkyl group. Another way of classifying Sure Sol®-125 is to say that it contains about 80 mole % methyl groups (based on total moles of alkyl groups), 15 mole % ethyl groups, and only 4 mole % propyl groups. Thus, Sure Sol®-125 does not contain many alkyl groups which can transalkylate onto biphenyl under the reaction conditions and cause impurity problems.

GC analyses were done using the aforementioned GC conditions.

## Synthesis of LOBBP(60):

Molten Koch 95% biphenyl (154.3g, 1.00 mole) was added to 15.7g of Sure Sol®-290. To the mixture was added 4.5g of an aluminum chloride catalyst complex made using Sure Sol®-125 as the aromatic hydrocarbon and HCl gas as the source of halide. The clear red solution (62°C) was sparged with 1-butene as it was cooled in ice water. When solids formed, the ice bath was removed to allow the reaction temperature to warm and melt the solids. The ice bath was then reapplied. As the butene addition proceeded, the temperature was lowered to 46°C, then to 40°C, then to 35°C. A total of 58.3g of butene (1.04 mole) was added over 45 minutes, following the course of the reaction by periodically weighing the apparatus and taking samples of the reaction mixtures. When quenched into water, the samples were almost colorless.

An additional 4.5g of catalyst complex was added for transalkylation. The temperature dropped from 35°C to 24°C. The reaction was quenched after 1 hr 40 minutes of transalkylation. GC analysis showed less than 1% IBBP content in the alkylate, most of it from the regular Sure Sol®-290 which was used as a diluent. The alkylate was distilled, removing the front end and taking the rest of the alkylate overhead for use as a diluent in a subsequent scale-up.

A mixture of 2009g (13.03 mole) Koch 95% biphenyl and 168.6g of the distilled low temperature alkylate prepared above was treated with 61.1g of AlCl$_3$/Sure Sol®-125/ methyl chloride catalyst complex followed by sparging with 616g butene over 2 hr 12 minutes. The temperature of the ice water cooled reaction dropped from 70°C to about 50°C at the halfway point to 42°C as the last of the butene was added. After the addition was complete another 67.3g of catalyst complex was added for the transalkylation. The temperature dropped to 23°C over the next 3 hr.

A 23.4g sample was withdrawn from the transalkylate. The small sample was heated to 120°C for 1 hr, then was quenched into aqueous sodium hydroxide.

The large-scale transalkylate was also quenched with aqueous sodium hydroxide. Both reaction products were analyzed for IBBP content by GC. The low temperature transalkylate had 0.066% 3-IBBP and 0.047% 4-IBBP (total 0.133%) while the high temperature transalkylate had 4.833% 3-IBBP and 3.637% 4-IBBP (total 8.470%). Raising the temperature of the transalkylation by 90°C produced 80 times more IBBP.

The transalkylate from the large scale reaction was distilled using a 35 theoretical stage distillation column packed with Heli-Pak® B packing at 100 mm Hg. Due to air leakage into the system most of the fractions had a "burnt" odor. A blend of fractions which represented a 43% distillation yield was made and passed through a bed of Floridan® clay to remove the burnt odor. Odor panel results showed that this blend had a lower, less intense odor than regular Sure Sol®-290.

A second batch of LOBBP(60) was made, using some of the LOBBP(60) blend from the first batch as the diluent in the alkylation. Table 1 contains the properties of the two blends.

Table 1

| Physical Properties of LOBBP Compared to Normal Sure Sol®-290 | | | |
|---|---|---|---|
| Property | Sure Sol®-290 | LOBBP(60), Batch #1 | LOBBP(60), Batch #2 |
| MAP, °F (°C) | 54.0 (12.2) | 55.5 (13.1) | 52.5 (11.4) |
| K. Vis., 100°F, cSt | 7.20 | 7.01 | 7.08 |
| S.G.(60°F/60°F) | 0.9725 | 0.9762 | 0.9771 |
| R.I., 25°C | 1.5709 | 1.5724 | 1.5731 |
| Pour point, °F | -57 | not determined | -65 |

Table 2 compares the compositions of regular Sure Sol®-290 with the composition of the first batch of LOBBP(60).

Table 2

| Comparative GC Composition of Sure Sol®-290 and LOBBP(60) | | |
|---|---|---|
| Component Region | Normal Sure Sol®-290 | LOBBP(60), Batch #1 |
| Lights region | 0.00 | 0.00 |
| Biphenyl | 0.00 | 0.00 |
| Betweens-1 region | 3.46 | 0.07 |
| SBBP region | 79.26 | 85.51 |
| Betweens-2 region | 2.17 | 1.75 |
| DSBBP region | 14.41 | 12.53 |
| Heavies region | 0.70 | 0.13 |

In these analyses, the GC chromatogram was divided into regions according to retention time. Note that the SBBP component region includes the IBBP isomers and the di-sec-butylbiphenyl (DSBBP) region includes some of the diisobutylbiphenyl (DIBBP) and the isobutyl-sec-butylbiphenyl (IBSBBP) isomers.

As shown in Table 1, the physical properties of the two LOBBP(60) blends are very similar to the physical properties of regular Sure Sol®-290. In Table 2, it can be seen that the compositions are very similar except that the normal Sure Sol®-290 betweens-1 region is larger than the betweens-1 region of the LOBBP(60). This is partially due to the higher temperature at which the regular Sure Sol®-290 is produced, making more impurities, and it is partially due to the use of aromatic hydrocarbons in the catalyst complex which make distillation of the regular Sure Sol®-290 more difficult, leading to inclusion of more impurities in order to achieve higher yields of product.

### Low Temperature Transalkylation of DSBBP and Biphenyl

It is important, in order to achieve the highest possible yields of product, to be able to recycle the heavier components of the alkylate such as the part of the DSBBP which is not used in the LOBBP product. Several fractions from the back end of the LOBBP(60) distillation were combined to make a DSBBP blend of 97% purity. A mixture of 40g of the DSBBP blend and 27.8g of molten 97% biphenyl (also from the distillation of the LOBBP(60)) was treated with 1.4g of AlCl₃/Sure Sol®-125/HCl catalyst complex. The mole ratio of butyl groups:total biphenyl (or AAG) was 0.91:1.0.

The reaction mixture was stirred as it cooled from 45°C. When the temperature dropped to 27°C the mixture solidified. It was heated to 32°C then to 52°C over the next few hours. It was held at 52°C overnight, for a total reaction time of 29 hours. After quenching, GC analysis showed that there was no detectable IBBP in the alkylate. Table 3 contains GC analysis information.

Table 3

| Transalkylation of Biphenyl and DSBBP at 52°C Catalyzed by AlCl$_3$ GC Analysis (area %) | | |
| --- | --- | --- |
| Component | Before adding cat. complex | After 29 hr of reaction |
| Lights region | 0.46 | 1.33 |
| Biphenyl | 39.06 | 22.69 |
| Betweens-1 region | 0.04 | 0.87 |
| SBBP region | 0.00 | 45.19 |
| Betweens-2 region | 0.12 | 0.69 |
| DSBBP region | 58.89 | 25.39 |
| Heavies region | 1.43 | 3.84 |

As can be seen in Table 3, the transalkylation at low temperature produces an acceptable amount of SBBP and the transalkylate would serve as a good source of LOBBP(50) product.

### LOBBP Made Using Vektor® 70 FCC Catalyst

Vektor® 70 FCC catalyst is made by Engelhard and is an ultrastable Y (USY) acidic zeolite used in fluid catalytic cracking (FCC) units in refineries. The zeolite is supported on an inert support. Before use in the present reaction, the catalyst was calcined at 550°C to remove most of the water contained in the catalyst.

Molten biphenyl containing 10% by weight Vektor® catalyst was heated at 180°C and 0.385 mole equivalent 1-butene was sparged into the red reaction mixture. The mixture was then filtered to remove the catalyst and the excess biphenyl was distilled away using a 35 theoretical stage column packed with Heli-Pak® B. The alkylate remaining in the still was drained and was short-path distilled to remove the yellow color and a slight off-odor which had developed. The product had a viscosity of 7.25 cSt at 100°F and contained 84% SBBP region and 12% DSBBP region. GC analysis indicated 0% 3-IBBP and 1.218% 4-IBBP. There were fewer minor impurities in the LOBBP(vektor®) than in regular Sure Sol®-290. Blind odor panel testing indicated this LOBBP(Vektor®) had a substantially lower, less intense odor than regular Sure Sol®-290.

### Transalkylation Using Vektor® Catalyst

A mixture of 26.9g 99% biphenyl and 36.8g of DSBBP blend (both from the LOBBP(60) distillation) was transalkylated using 5.9g of Vektor® 70 catalyst at 180°C for 5 hr. Table 4 contains the results of GC analysis of the filtered transalkylate.

Table 4

| Transalkylation of Biphenyl and DSBBP at 180°C Catalyzed by Vektor® 70; GC Area % Analysis | |
| --- | --- |
| Component | After 5 hr of reaction |
| Lights region | 0.27 |
| Biphenyl | 24.95 |
| Betweens-1 region | 1.29 |
| SBBP region | 44.84 |
| Betweens-2 region | 1.04 |
| DSBBP region | 24.2 |
| Heavies region | 3.42 |

Again, an acceptable amount of SBBP was produced and the transalkylate would serve to make LOBBP(Vektor®) product.

## LOBBP at 80°C and 100°C

The synthesis of LOBBP was examined at 80°C and 100°C to determine the upper limit for production of low quantities of IBBP. Two small scale alkylations were run, one at 80°C (maximum temperature 88°C) and one at 100°C (maximum temperature 101°C) using the $AlCl_3$/Sure Sol®-125/methyl chloride catalyst complex and adding about 0.70 mole equivalent butene. Since both reactions were run above the melting point of Koch 95% biphenyl, there was no need for a diluent to prevent biphenyl crystallization. Each reaction was transalkylated at either 80°C or 100°C using a second dose of catalyst complex. After quenching, GC analysis was done. Table 5 contains the results of the analyses.

Table 5

| SBBP and IBBP Conn tent of LOBBP Alkylates and Products, GC Area % Normalized | | | | |
|---|---|---|---|---|
| Sample Analyzed, Conditions | 3-SBBP | 3-IBBP | 4-IBBP | 4-SBBP |
| LOBBP(60) product | 23.53% | 0.08% | 0.00% | 76.39% |
| LOBBP(80) alkylate | 61.29% | 1.10% | 1.02% | 36.59% |
| LOBBP(Vektor®) product | 51.40% | 1.56% | 1.30% | 45.74% |
| LOBBP(100) alkylate | 56.08% | 6.27% | 4.67% | 32.98% |
| Sure Sol®-290 product, 120+°C | 54.96% | 6.01% | 6.39% | 32.64% |

From Table 5 it can be seen that conducting the alkylation at 100°C produces an alkylate which contains almost as much IBBP isomers as the regular Sure Sol®-290 product which is produced at 120+°C. Lowering the temperature to 80°C produces an alkylate which has a much lower amount of IBBP isomers.

## Scale-up of LOBBP(80) Synthesis

The synthesis of LOBBP(80) was scaled up to produce enough material for a distillation and blend production. Molten biphenyl was alkylated at 80°C using the $AlCl_3$/ Sure Sol®-125/methyl chloride catalyst complex, adding about 0.70 mole equivalent butene. The reaction mixture was transalkylated with a second dose of the same catalyst complex. Distillation and blending of certain fractions gave an LOBBP(80) product with the following properties as detailed in Table 6.

Table 6

| Physical Properties of LOBBP(60) and LOBBP(80) Compared to Normal Sure Sol®-290 | | | |
|---|---|---|---|
| Property | Sure Sol®-290 | LOBBP(60), Batch #2 | LOBBP(80) |
| MAP, °F (°C) | 54.0 (12.2) | 52.5 (11.4) | 54.0 (12.2) |
| K. Vis., 100°F, cSt | 7.20 | 7.08 | 7.43 |
| S.G.(60°F/60°F) | 0.9725 | 0.9771 | 0.9751 |
| R.I., 25°C | 1.5709 | 1.5731 | 1.5706 |
| Pour point, °F | -57 | -65 | not determined |

The LOBBP(80) had properties very similar to the properties of regular Sure Sol®-290 and LOBBP(60).

It was noticed that regular Sure Sol®-290 had over 3% of the betweens-1 region content while earlier LOBBP(60) blends had less than 1% betweens-1 content. Since the betweens-1 content can influence the odor of the blends, an LOBBP(80) blend was made with as much betweens-1 content as possible without increasing the biphenyl content past 0.10%. Table 7 compares the betweens-1 contents and the individual isomer content of several of the blends.

Table 7

| Betweens-1, SBBP, and IBBP Isomer Content of Sure Sol®-290 Products, GC Area % | | | | | |
|---|---|---|---|---|---|
| Product | Betweens-1 | 3-SBBP | 3-IBBP | 4-IBBP | 4-SBBP |
| LOBBP(60) | 0.070 | 19.607 | 0.071 | 0.000 | 63.655 |
| LOBBP(80) | 1.010 | 49.462 | 0.872 | 0.712 | 30.198 |
| LOBBP(Vektor®) | 0.245 | 43.566 | 1.322 | 1.099 | 38.766 |
| Sure Sol®-290 | 3.560 | 44.352 | 4.854 | 5.157 | 26.339 |

Odor panel testing of the LOBBP(80) showed that it had a lower, less intense odor than regular Sure Sol®-290 even with its relatively high betweens-1 content.

The ratios of the 3-SBBP and 4-SBBP isomers in the various products was determined by GC analysis. Table 8 contains the results of these analyses.

Table 8

| Ratio of SBBP Isomers in the Products, GC Area % Normalized | | | |
|---|---|---|---|
| Product, Conditions | 3-SBBP | 4-SBBP | Ratio |
| LOBBP(60) product | 23.53% | 76.39% | 1.00:3.25 |
| LOBBP(80) alkylate | 61.29% | 36.59% | 1.68:1.00 |
| LOBBP(Vektor®) product | 51.40% | 45.74% | 1.12:1.00 |
| LOBBP(100) alkylate | 56.08% | 32.98% | 1.70:1.00 |
| Sure Sol®-290 | 54.96% | 32.64% | 1.68:1.00 |

Note that the LOBBP(60) contains more 4-SBBP than 3-SBBP and has an isomer ratio which is reversed when compared to the other products. While LOBBP(60) has a lower, less intense odor than regular Sure Sol®-290, it is also a "different" odor; some have described it as a "sweeter" odor than regular Sure Sol®-290. It would be best if any new product had a lower but similar odor to regular Sure Sol®-290 in order to serve as a replacement for Sure Sol®-290. The ratio of 3-SBBP to 4-SBBP in the LOBBP(60) may be one of the reasons for its perceived different odor. Without wishing to be limited by any theory, LOBBP products with a 3-SBBP:4-SBBP ratios similar to regular Sure Sol®-290 are preferable, and thus the LOBBP(80) is a preferable product for replacement of Sure Sol®-290 in the marketplace.

**Transalkylation of DSBBP at 80°C**

As previously mentioned, in order to have an economical process, one must be able to use the DSBBP which is produced in the alkylation but is not subsequently used in the LOBBP(80) product. A 1:1 weight ratio of biphenyl:DSBBP was transalkylated at 80°C with the $AlCl_3$/Sure Sol®-125/methyl chloride catalyst complex. Tables 9 and 10 following contain details of the composition of the transalkylate.

Table 9

| Transalkylation of DSBBP and Biphenyl at 80°C, GC Area % Regions | | | | | |
|---|---|---|---|---|---|
| Time (min) | Biphenyl | SBBP | DSBBP | TSBBP | Total |
| 0 | 49.05 | 0.02 | 50.43 | 0.03 | 99.54 |
| 15 | 28.19 | 40.30 | 25.17 | 2.84 | 96.49 |
| 30 | 25.75 | 44.98 | 22.88 | 2.47 | 96.08 |
| 45 | 24.80 | 45.49 | 23.07 | 2.48 | 95.84 |
| 60 | 25.05 | 45.48 | 22.71 | 2.45 | 95.68 |

Note that in Table 9, only 4.5% of the components of the transalkylate fall outside of the major regions after 60 minutes. A thermodynamic equilibrium was reached after 30 minutes of transalkylation, and the maximum amount of SBBP produced was higher (45%) than in the 80°C alkylation (43%).

Table 10

| Transalkylation of DSBBP and Biphenyl at 80°C, Normalized GC Area % of Isomers | | | | |
|---|---|---|---|---|
| Time (min) | 3-SBBP | 3-IBBP | 4-IBBP | 4-SBBP |
| 0 | 0.00 | 0.00 | 0.00 | 0.00 |
| 15 | 59.18 | 0.86 | 0.64 | 39.32 |
| 30 | 61.46 | 1.09 | 0.87 | 36.58 |
| 45 | 61.47 | 1.25 | 0.94 | 36.35 |
| 60 | 61.58 | 1.17 | 0.92 | 36.32 |

In Table 10, the amount of IBBP isomers produced was very low, lower than that produced in an 80°C alkylation.

Based on the preceding work, it was concluded that DSBBP from an 80°C alkylation can be transalkylated to produce an acceptable LOBBP(80) feedstock.

### Alkylation Curves for Regular Sure Sol®-290 and LOBBP(80)

One way to optimize a process which involves alkylation is to construct alkylation curves in which the composition of the alkylate is determined at various mole ratios of butene:biphenyl. In that way, the mole ratio butene:biphenyl at which the yield is maximized can be determined.

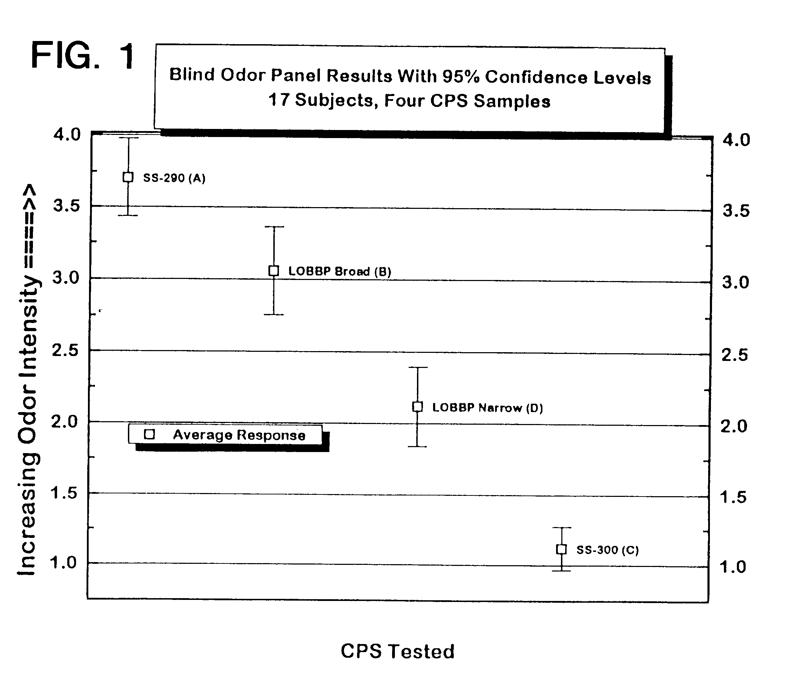

A series of lab alkylations were carried out both at 80°C and at 120°C, adding various mole ratios butene:biphenyl with an $AlCl_3$/Sure Sol®-125/methyl chloride catalyst complex. Each alkylation was individually treated with a second dose of catalyst complex to insure complete transalkylation to the thermodynamic equilibrium composition characteristic of that particular mole ratio butene:biphenyl and temperature. The alkylation curves produced at 120°C as shown in Fig. 1.

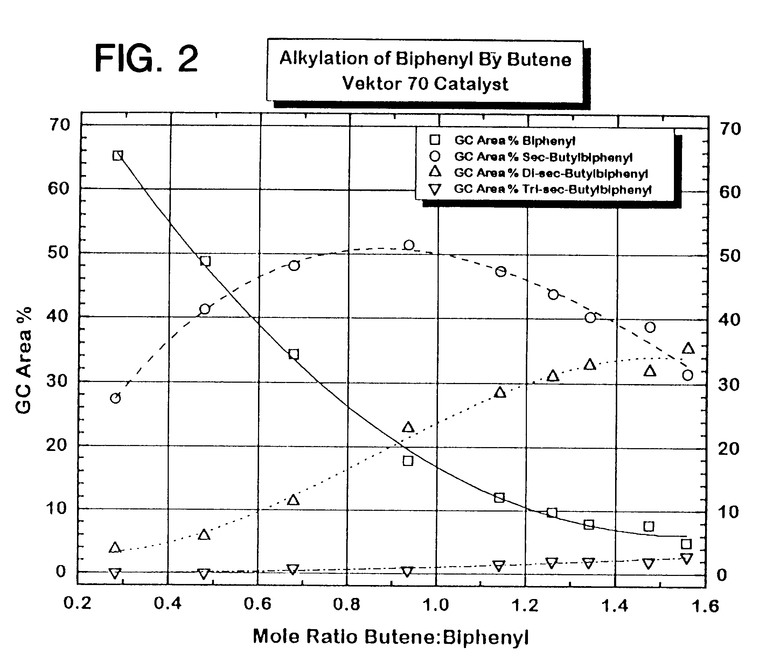

Note that the maximum SBBP concentration is about 41% at about 0.9 mole ratio butene:biphenyl. Fig. 2 contains the results of determining alkylation curves at 80°C.

Note that in Fig. 2 the maximum SBBP concentration is about 43% at about 0.85 mole ratio butene:biphenyl. We can therefore obtain a higher yield of product using the inventive reaction process conditions compared to the reaction process conditions of the prior art.

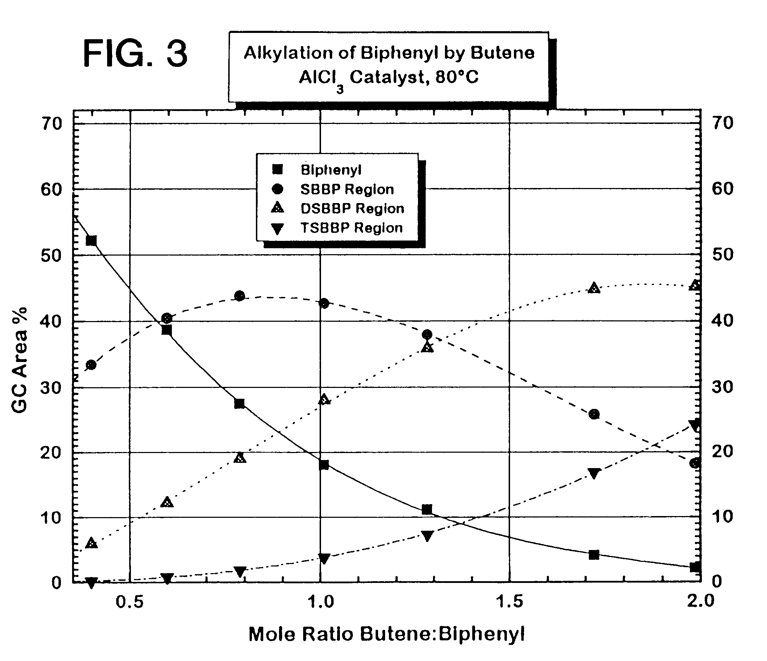

Fig. 3 is similarly constructed using the data from the Vektor 70 catalyzed reaction. Note that the maximum concentration of SBBP is over 50%. Again, the yield of LOBBP product from the inventive process is higher than the yield of product from the process of the prior art.

### Synthesis and Purification of Authentic 4-IBBP

The synthesis of 4-IBBP is known in the art. Biphenyl was first acylated with isobutyryl chloride using a full equivalent of $AlCl_3$ as catalyst in ethylene dichloride solvent below 20°C. The ketone product was short path distilled to separate it from unreacted biphenyl and from heavier by-products. The yield was about 73%.

The ketone oxygen was removed using the classical Wang-Minlon modification of the Wolf-Kishner reduction in diethylene glycol with excess KOH and hydrazine hydrate. The product was fractionally distilled. A blend of 4-IBBP was made from fractions which were greater than 99% pure. The blend had an odor reminiscent of hydrazine hydrate, apparently due to impurities produced by cracking of nitrogenous heavy components at the high temperatures of the fractional distillation. The blend was taken up in hexane and was washed at reflux for a short time with 2N HCl. The acid wash was repeated at room temperature then the organic layer was filtered through a bed of Floridan® clay to insure removal of all polar nitrogenous compounds. The filtrate was concentrated in vacuo then short path distilled to remove all traces of hexane. The product was 100% by GC analysis.

From the LOBBP(60) distillations one fraction contained 80% pure 3-SBBP and one fraction contained 98% pure 4-SBBP. Each fraction was filtered through Floridan® clay to remove a burnt odor due to the presence of oxygen during the distillation.

A blend of 90:10: :4-SBBP:4-IBBP was made in order to simulate the ratio of SBBP:IBBP in regular Sure Sol®-290. Blind odor panel analysis found that the 90:10 blend of 4-SBBP:4-IBBP had a higher, more intense odor than the pure 98% 4-SBBP, thus establishing that the IBBP is one reason for the higher odor of regular Sure Sol®-290.

**Plant Trial Production of LOBBP(80)**

Using a 4000 gallon glass-lined reactor, 2500 gallons of molten biphenyl containing 40 gallons of $AlCl_3$/cumene/HCl catalyst complex was alkylated with 1000 gallons of 88% pure butene over a 6 hour cycle time in a plant trial production of LOBBP(80). The average temperature of the reaction mixture was 82°C. For the transalkylation, the mixture was stirred for 45 minutes after the butene addition was completed. No additional catalyst dose was added.

After quenching, a small portion of the alkylate was lab distilled. Two blends were made from the lab distillation.

Table 11 details the IBBP content of the plant trial. Because the average temperature of the plant trial batch was a little higher than the lab alkylations, the IBBP content of the alkylate was a little higher.

Table 11

| SBBP and IBBP Content of Sure Sol®-290 Alkylates and Products, GC Area % Normalized | | | | |
|---|---|---|---|---|
| **Product, Conditions** | **3-SBBP** | **3-IBBP** | **4-IBBP** | **4-SBBP** |
| LOBBP(80) alkylate, lab | 61.29% | 1.10% | 1.02% | 36.59% |
| LOBBP(80) alkylate, plant trial | 60.27% | 1.39% | 1.57% | 36.77% |

Also, since the cumene catalyst complex was used in the plant trial alkylation, the alkylate contained isopropylbiphenyl (IPBP) impurities, as shown in Table 12. The IPBP impurities have retention times which put them into the betweens-1 region. The column in Table 12 labeled "(IPBP)" shows the amount of IPBP in the betweens-1 region in both the lab LOBBP(80) alkylate and the plant trial LOBBP(80) alkylate. When the cumene catalyst complex was used, the alkylate had 20 times more IPBP in it versus the amount of IPBP present in the lab alkylate when the Sure Sol®-125 catalyst complex was used.

Table 12

| Composition of LOBBP Alkylates, GC Area % | | | | | | | | | | |
|---|---|---|---|---|---|---|---|---|---|---|
| **Alkylate** | **Lites** | **BP** | **Bet-1** | **(IPBP)** | **SBBP** | **Bet-2** | **DSBBP** | **Bet-3** | **TSBBP** | **Hvy** |
| LOBBP(80), lab, 0.60 mole ratio | 3.93 | 38.73 | 2.09 | (0.12) | 40.51 | 1.24 | 12.16 | 0.04 | 0.81 | 0.50 |
| LOBBP(80), plant trial | 2.38 | 37.3 | 4.05 | (2.67) | 39.92 | 2.09 | 12.90 | 0.17 | 0.95 | 0.23 |

This amount of IPBP made it more difficult to distill the LOBBP(80) alkylate from the plant trial and make a low-odor product. After the lab distillation of the alkylate, two blends were made with the compositions as shown in Table 13.

Table 13

| Composition of LOBBP(80) Blends, GC Area %, Compared to Composition of Sure Sol®-290 | | | | | | | | |
|---|---|---|---|---|---|---|---|---|
| **Product** | **Lites** | **Biphenyl** | **Bet-1** | **SBBP** | **(IBBP)** | **Bet-2** | **DSBBP** | **Heavier** |
| LOBBP(80), plant trial, narrow blend | 0.01 | 0.02 | 0.20 | 81.95 | (1.90) | 6.71 | 11.11 | 0.00 |
| LOBBP(80), plant trial, broad blend | 0.03 | 0.07 | 4.14 | 80.46 | (1.90) | 4.24 | 11.05 | 0.02 |
| LOBBP(80), lab blend | 0.00 | 0.04 | 1.01 | 81.24 | (1.58) | 3.12 | 14.59 | 0.00 |
| Sure Sol®-290 | 0.00 | 0.06 | 3.56 | 80.75 | (12.40) | 3.22 | 12.41 | 0.01 |

One blend, the "LOBBP(80) narrow blend" contained 0.20% of the betweens-1 components. The other blend, the "LOBBP(80) broad blend" contained 4.14% of the betweens-1 components. These are contrasted with the lab blend of LOBBP(80), which had 1.01% betweens-1 components and regular Sure Sol®-290, which has 3.56% betweens-1 in it. All three of the LOBBP(80) blends had much lower IBBP content than regular Sure Sol®-290.

The two LOBBP(80) blends from the plant trial were compared to regular Sure Sol®-290 and to Sure Sol®-300, a very low odor carbonless paper solvent in Table 14.

## Table 14

## Blind Odor Panel Testing of LOBBP(80) Plant Trial Blends

Strongest Odor = 4
Weakest Odor = 1

| Subject | SS-290(A) | LOBBP Broad (B) | LOBBP Narrow (D) | SS-300(C) |
|---|---|---|---|---|
| 1 | 4 | 3 | 2 | 1 |
| 2 | 4 | 3 | 2 | 1 |
| 3 | 4 | 3 | 1 | 2 |
| 4 | 4 | 2 | 3 | 1 |
| 5 | 4 | 3 | 2 | 1 |
| 6 | 4 | 3 | 2 | 1 |
| 7 | 4 | 3 | 2 | 1 |
| 8 | 4 | 3 | 1 | 2 |
| 9 | 4 | 3 | 2 | 1 |
| 10 | 4 | 3 | 2 | 1 |
| 11 | 4 | 3 | 2 | 1 |
| 12 | 2 | 4 | 3 | 1 |
| 13 | 4 | 2 | 3 | 1 |
| 14 | 3 | 4 | 2 | 1 |
| 15 | 4 | 2 | 3 | 1 |
| 16 | 3 | 4 | 2 | 1 |
| 17 | 3 | 4 | 2 | 1 |
| Average | 3.71 | 3.06 | 2.12 | 1.12 |
| Standard Deviation | 0.570 | 0.639 | 0.582 | 0.322 |
| 95% Confidence Level | 0.27± | 0.30± | 0.28± | 0.15± |

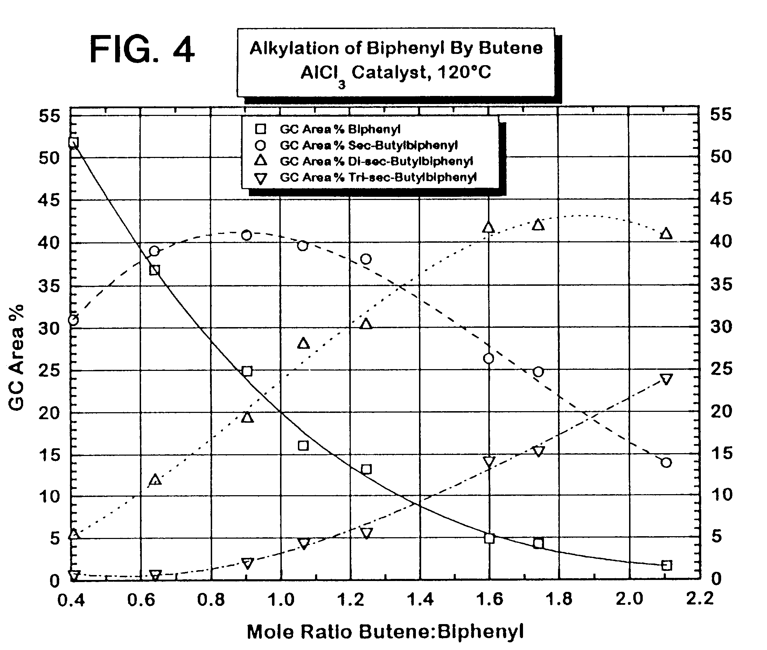

Based on a statistical analysis of the odor rankings, it was determined with 95% confidence level that regular Sure Sol®-290 had the highest odor and the LOBBP(80) narrow blend had a very low odor, not as low as Sure Sol®-300, but clearly differentiated from the Sure Sol®-290 and from the LOBBP(80) broad blend. Odor panel participants, even though the LOBBP(80) broad blend had more of the betweens-1 components than regular Sure Sol®-290, were able to differentiate it from regular Sure Sol®-290, and were able to say with a 95% confidence level that the LOBBP(80) broad blend had a lower odor than regular Sure Sol®-290. This is illustrated in Fig. 4, where the differences between the samples is statistically significant.

Although not wishing to be limited by any theory, the lower IBBP content of the LOBBP(80) broad blend is what differentiated its odor from that of regular Sure Sol®-290.

## Transalkylation of Alkylate Produced Using a Clay Catalyst

Biphenyl was alkylated with 0.943 mole equivalent butene at 120-130°C in the presence of 10 wt % Harshaw-Filtrol® 13LM. After the butene addition the alkylate was filtered to remove the catalyst and the filtrate was distilled using the 35 theoretical stage column. A blend of fractions was made; the blend had the composition as shown in the first row of Table 15 and was transalkylated using an Sure Sol®-125/AlCl₃/methyl chloride catalyst complex at 80°C. The results are shown in Table 15.

Table 15

| Transalkylation of Clay Catalyzed Butylbiphenyl Still Bottoms, GC Area % Composition | | | | | | | | | |
|---|---|---|---|---|---|---|---|---|---|
| Time, min | Lites | Biphenyl | Bet-1 | SPBP | Bet-2 | DSBBP | Bet-3 | TSBBP | Heavies |
| 0 | 0.01 | 0.02 | 1.19 | 35.11 | 40.09 2 | 13.00 | 5.27 | 2.50 | 1.99 |
| 20 | 2.02 | 3.83 | 0.25 | 29.41 | 1.12 | 46.93 | 0.53 | 14.38 | 1.52 |
| 47 | 2.08 | 4.67 | 0.31 | 27.74 | 1.42 | 45.62 | 0.76 | 15.67 | 1.74 |
| 70 | 2.04 | 4.64 | 0.41 | 27.89 | 1.23 | 45.44 | 0.46 | 16.07 | 1.83 |

In Table 15, note that the Bet-1 and Bet-2 regions (in which fall the ortho-rich sec-butylbiphenyl and di-sec-butylbiphenyl) were already greatly reduced in 20 minutes, an indication of how quickly the transalkylation is completed. The transalkylation produced very small amounts of IBBP; no 3-IBBP was detected in any of the samples and the maximum amount of 4-IBBP detected was 0.10%.

In a second experiment a portion of the blend was mixed with biphenyl such that the AAG content after mixing was 0.72 (equivalent to the alkylation of biphenyl with butene to a mole ratio butene:biphenyl of 0.72). This mixture was transalkylated and the product was analyzed. The results are in Table 16 following.

Table 16

| Transalkylation of Clay Catalyzed Butylbiphenyl Still Bottoms + Biphenyl, 0.72 Average Alkyl GC Area % Composition | | | | | | | | | |
|---|---|---|---|---|---|---|---|---|---|
| Time, min | Lites | Biphenyl | Bet-1 | SBBP | Bet-2 | DSBBP | Bet-3 | TSBBP | Heavies |
| 0 | 0.01 | 50.71 | 0.67 | 18.98 | 20.16 | 6.24 | 2.46 | 0.27 | 0.51 |
| 15 | 1.83 | 34.53 | 0.69 | 44.10 | 0.54 | 16.58 | 0.12 | 1.34 | 0.26 |
| 36 | 1.60 | 32.82 | 0.77 | 45.28 | 0.66 | 17.34 | 0.09 | 1.41 | 0.03 |
| 69 | 1.56 | 31.10 | 0.91 | 46.01 | 0.67 | 17.66 | 0.03 | 1.53 | 0.51 |

Again, the transalkylation was essentially over in 15 minutes. The yield of SBBP at that time was over 44%, greater than the yield of SBBP in a normal alkylation. Allowing the transalkylation to proceed for a little over an hour produced 46% SBBP. As with the previous transalkylation, no 3-IBBP was detected and the maximum amount of 4-IBBP detected was 0.10%.

## Synthesis of DSBBP

By comparison of Figs. 1 and 2 it can be seen that the process of the present invention produces a higher concentration of DSBBP than does the prior art process. The highest concentration of DSBBP produced by alkylation of biphenyl by butene at 120+°C is about 43% while the highest concentration of DSBBP produced by alkylation of biphenyl by butene at 80°C is 45%. The DSBBP produced at 80°C has fewer minor impurities also.

Molten biphenyl is alkylated by 1.8 mole equivalent butene at 80°C using the AlCl₃/ Sure Sol®-125/methyl chloride catalyst complex. After a 1 hr transalkylation, catalyzed by a second dose of catalyst, the reaction is quenched and the alkylate is distilled. Fractions containing a high purity of DSBBP are combined to give DSBBP with acceptable physical properties and a very low odor.

## Preparation of Carbonless Paper System

To 100 parts LOBBP (80) is added 8 parts leuco black dye of the alkylamino-fluorane type. The mixture is warmed to 100°F and agitated to give completed solution. To the resultant solution is added 100 parts of mixed triisopropyltoluene isomers (TIPT) and the entire solution allowed to equilibrate. This solution, added to 150 parts aqueous solution containing 35 parts gum arabic, gives on agitation a stable suspension which, upon addition of 200 parts of a 12% gelatine solution, sufficient sodium hydroxide to maintain the pH at 9 and additional water (800 parts) gives a suspension which, upon further addition of acetic acid to pH=4 to 4.5 under agitation yields a suspension of oil microdroplets. Further addition of formaldehyde solution (4 parts $CH_2O$) and subsequent adjustment of pH to 9.65 causes hardening of the microdroplets to capsules. These microcapsules when applied by standard techniques to paper at a rate of about 5 gm/m$^2$, and dried, produced paper ready for acid development by clays or by acid resins, when broken by pressure from a stylus.

## Claims

1. A process for the production of *sec*-butylbiphenyl and/or di-*sec*-butylbiphenyl, comprising contacting butene and biphenyl under alkylation conditions, in the presence of a catalyst complex comprising a Lewis acid, an aromatic hydrocarbon and a hydrogen halide or alkyl halide, wherein more than 50 mole % of the alkyl group substituents on the aromatic hydrocarbon are methyl groups.

2. The process of claim 1, which is conducted at 0 to 115°C, preferably 25 to 100°C.

3. The process of claim 1 or claim 2, wherein the Lewis acid is selected from aluminium chloride, aluminium bromide, tin chloride, titanium chloride and boron trifluoride, preferably aluminium chloride.

4. The process of claim 3, wherein the Lewis acid comprises aluminium chloride and the hydrogen halide or alkyl halide comprises methyl chloride.

5. The process of any preceding claim, wherein more than 70 mole %, and preferably more than 80 mole %, of the alkyl group substituents on the aromatic hydrocarbon are methyl groups.

6. The process of any preceding claim, wherein the aromatic hydrocarbon comprises one or more alkyl benzenes or polyalkylbenzenes, e.g. one or more of isopropylbenzenes, xylenes, trimethylbenzenes, tetramethylbenzenes, ethylbenzenes, diethylbenzenes, triethylbenzenes, dimethylethylbenzenes, diisopropylbenzenes and triisopropylbenzenes.

7. The process of any preceding claim, wherein the mole ratio of Lewis acid:aromatic hydrocarbon:hydrogen halide or alkyl halide in the catalyst complex is approximately 2:1:0.5.

8. The process of any preceding claim, wherein the catalyst complex is present in an amount of from 0.01 to 10 wt%, based on the weight of the Lewis acid and the biphenyl.

9. The process of any preceding claim, wherein a non-reactive or reactive diluent is added to the butene and/or to the biphenyl.

10. The process of any preceding claim, including subjecting the product to transalkylation conditions effective to bring the product to thermodynamic equilibrium.

11. The process of claim 10, wherein the transalkylation conditions include adding additional amounts of the catalyst complex to the product or comprise holding the product under the alkylation conditions.

12. A process for the production of *sec*-butylbiphenyl and/or di-*sec*-butylbiphenyl, comprising contacting butene and biphenyl in the presence of a zeolite catalyst selected from Y zeolites, USY zeolites, mordenite zeolites, ZSM-12 zeolites, and FCC catalysts.

13. The process of claim 12, which is conducted at 50 to 300°C, preferably 120-260°C.

**14.** The process of claim 12 or claim 13, wherein the zeolite catalyst is a USY zeolite.

**15.** The process of any of claims 12 to 14, wherein the zeolite catalyst is present in an amount of 1 to 20 wt%, preferably 5 to 15 wt%, based on the weight of the biphenyl.

**16.** The process of any preceding claim, wherein the butene is 1-butene.

**17.** The process of any of claims 1 to 15, wherein the butene is *cis-* or *trans*-2-butene.

**18.** The process of any preceding claim, wherein the butene is present in amount from 0.2 to 2.6 mole equivalent, preferably 1.0 mole equivalent, based on the biphenyl.

**19.** A process for the production of *sec*-butylbiphenyl and/or di-*sec*-butylbiphenyl, comprising contacting a feedstock containing two or more of biphenyl, *sec*-butylbiphenyl, di-*sec*-butylbiphenyl and tri-*sec*-butylbiphenyl, under transalkylation conditions effective to transfer *sec*-butyl groups between the components of the feedstock, in the presence of a catalyst complex as defined in claim 1, or a zeolite catalyst as defined in claim 12.

**20.** Microcapsules suitable for use in carbonless paper system, the microcapsules containing a low odour solvent comprising about 50 to 100% by wt *sec*-butylbiphenyl, about 0 to 45 % by wt di-*sec*-butylbiphenyl, and less than about 10% by wt isobutylbiphenyl; and a dye solubilised in the solvent.

FIG. 1

Blind Odor Panel Results With 95% Confidence Levels
17 Subjects, Four CPS Samples

EP 0 819 667 A2

21

FIG. 2

Alkylation of Biphenyl By Butene
Vektor 70 Catalyst

GC Area %

Mole Ratio Butene:Biphenyl

□ GC Area % Biphenyl
○ GC Area % Sec-Butylbiphenyl
△ GC Area% Di-sec-Butylbiphenyl
▽ GC Area % Tri-sec-Butylbiphenyl

FIG. 3

Alkylation of Biphenyl by Butene
AlCl$_3$ Catalyst, 80°C

GC Area %

Mole Ratio Butene:Biphenyl

■ Biphenyl
● SBBP Region
△ DSBBP Region
▼ TSBBP Region

FIG. 4

Alkylation of Biphenyl By Butene
$AlCl_3$ Catalyst, 120°C

Legend:
□ GC Area % Biphenyl
○ GC Area % Sec-Butylbiphenyl
△ GC Area % Di-sec-Butylbiphenyl
▽ GC Area % Tri-sec-Butylbiphenyl

Y-axis: GC Area %
X-axis: Mole Ratio Butene:Biphenyl

EP 0 819 667 A2

24